# FASCICULE DE BREVET EUROPEEN

(11) **EP 3 882 555 B1**
(45) Date de publication et mention de la délivrance du brevet: **06.11.2024**
(21) Numéro de dépôt: 21163520.6
(22) Date de dépôt: 18.03.2021
(51) Int. Cl.: F28D 20/00, A61L 2/04

(54) **UNITÉ DE VALORISATION, DISPOSITIF DE STÉRILISATION COMPRENANT L'UNITÉ DE VALORISATION ET PROCÉDÉ ASSOCIÉ**
AUFWERTUNGSEINHEIT, STERILISATIONSGERÄT, DAS DIESE AUFWERTUNGSEINHEIT UMFASST, UND ENTSPRECHENDES VERFAHREN
RECOVERY UNIT, STERILISATION DEVICE COMPRISING THE RECOVERY UNIT AND ASSOCIATED METHOD

(30) Priorité: 20.03.2020 FR 2002767
(43) Date de publication de la demande: 22.09.2021
(73) Titulaire: Commissariat à l'Energie Atomique et aux Energies Alternatives, 75015 Paris (FR)
(72) Inventeur: VESIN, Sébastien, 38054 GRENOBLE (FR); POUVREAU, Jérôme, 38054 GRENOBLE (FR)
(74) Mandataire: Hautier IP

(56) Documents cités:
- EP-A1- 1 906 101
- EP-A1- 4 081 750
- WO-A1-2021/130648
- WO-A1-82/04370
- FR-A1- 2 533 538
- US-B2- 9 566 356

## Description

### DOMAINE TECHNIQUE

La présente invention concerne le domaine de la récupération d'énergie et de l'optimisation de l'utilisation de l'énergie thermique. Elle trouve pour application particulièrement avantageuse le domaine des stérilisateurs utilisant de la chaleur de manière discontinue pour la mise en oeuvre d'un cycle de stérilisation.

### ÉTAT DE LA TECHNIQUE

L'optimisation écologique des appareils et procédés industriels est une démarche dans laquelle les industriels s'engagent de plus en plus. L'optimisation écologique, en plus d'avoir un effet positif sur l'environnement, contribue à l'image de l'entreprise. L'optimisation écologique passe couramment par une optimisation énergétique permettant une réduction des coûts.

Parmi les procédés industriels pouvant être optimisés, les dispositifs et procédés de stérilisation sont de grands consommateurs d'énergie et d'eau dont une majeure partie n'est pas recyclée. Un axe de recherche concerne le stockage de l'énergie fatale issue de ces procédés de stérilisation.

Une première solution de stockage thermique en apparence évidente et simple à mettre en place consisterait à remplir un réservoir avec l'eau chaude récupérée lors de la phase de refroidissement du stérilisateur, puis de restituer la chaleur de cette eau chaude stockée, lors de la phase de chauffage du cycle suivant. Dans cette configuration, l'eau chaude collectée est mélangée dans le stockage avec l'eau déjà présente conduisant à une homogénéisation progressive de sa température. Malgré sa simplicité, ce système présente une limite d'exploitation très pénalisante, puisqu'au cours de la phase de chauffage suivante du stérilisateur, la solution de stockage de chaleur ne pourra être exploitée seulement tant que la température de l'eau à sa sortie permet d'assurer le suivi du barème de l'eau de procédé alimentant le stérilisateur. Or l'homogénéisation de la température de l'eau lors de sa récupération et de son stockage limite grandement les possibilités d'exploitation de cette chaleur récupérée et conduit à considérer de grands volumes de stockage pour au final valoriser qu'une faible quantité d'énergie thermique.

On connait le document US 9,566,356 B2 décrivant un dispositif de stérilisation à récupération d'énergie.

Le dispositif de ce document comprend une chambre de stérilisation dans laquelle les produits à stériliser sont déposés. La chambre de stérilisation est reliée à un circuit de fluide primaire. Les produits à stériliser dans la chambre de stérilisation sont pulvérisés avec de l'eau chaude issue du circuit de fluide primaire. Dans ledit circuit de fluide primaire, le fluide est chauffé ou refroidi au moyen d'un échangeur de chaleur. Ce premier échangeur de chaleur comprenant à cet effet une entrée de vapeur, une conduite d'alimentation d'un agent de refroidissement, une conduite de retour de l'agent de refroidissement et un drain de condensat. Un second échangeur de chaleur est agencé en série sur le circuit de fluide primaire et est connecté à un circuit de fluide secondaire. Le deuxième échangeur permet de chauffer ou de refroidir le circuit de fluide primaire grâce à un circuit de fluide secondaire. Le dispositif comprend également un réservoir de stockage stratifié. Lors d'un cycle de refroidissement du circuit de fluide primaire, l'énergie du circuit de fluide primaire est donc transmise via le deuxième échangeur de chaleur au circuit de fluide secondaire et peut être stockée dans le réservoir de stockage stratifié. Lorsque le refroidissement par transfert thermique au circuit de fluide secondaire n'est plus possible alors le circuit de fluide primaire est refroidi via la conduite d'alimentation d'un agent de refroidissement dans le premier échangeur de chaleur jusqu'à ce que la température cible soit atteinte. Lors d'un prochain chauffage de la chambre, le fluide chaud est déstocké de manière stratifiée en température depuis le réservoir de stockage stratifié vers le circuit de fluide primaire via le deuxième échangeur de chaleur tant que le transfert de chaleur vers le circuit de fluide primaire est possible, ensuite le circuit de fluide primaire est chauffé via la conduite de vapeur dans le premier échangeur de chaleur jusqu'à ce que la température cible soit atteinte. Le réservoir de stockage stratifié est compartimenté physiquement de sorte à discrétiser le stockage du fluide secondaire en fonction de sa température dans les différents compartiments du réservoir. Le dispositif de l'état de la technique est complexe et peu compact.

On connait du document FR 2 533 538 un stockage d'eau chaude par stratification dans au moins un réservoir. Pour assurer la stratification de l'eau en fonction de sa température, le réservoir prévoit une conduite d'extraction/introduction d'eau chaude en partie supérieure et une conduite d'introduction/extraction d'eau froide en partie inférieure du réservoir. Ce document décrit le soutirage d'eau froide en partie basse pour la réchauffer au contact d'un échangeur et la réintroduire en partie supérieure et le soutirage d'eau chaude en partie haute et l'introduction d'eau froide en partie basse. Ce document concerne le stockage et l'utilisation d'un stockage d'eau par thermocline. Ce type de stockage n'est pas optimal pour la récupération et la réutilisation d'énergie thermique fatale.

Il existe donc le besoin d'améliorer la valorisation de l'énergie thermique récupérée et utilisée.

### RÉSUMÉ

Pour atteindre cet objectif, l'invention concerne une unité de valorisation selon la revendication 1. Cette unité comprenant un module de stockage de l'énergie thermique apte à recevoir un fluide de stockage, un échangeur thermique et un circuit de stockage thermique configuré pour relier fluidiquement le module de stockage et l'échangeur thermique et assurer la circulation du fluide de stockage, caractérisé en ce que:
le module de stockage comprend un volume vide dit volume tampon et en ce que le module de stockage d'énergie comprend au moins deux réservoirs de stockage de chaleur sensible, dont au moins un premier réservoir et un deuxième réservoir, le fluide de stockage destiné à être stocké dans le module de stockage représente un volume inférieur au volume du module de stockage de sorte à maintenir un volume du module de stockage vide dit volume tampon.

Le module de stockage comprend un système d'injection et de soutirage du fluide de stockage avantageusement agencé en partie inférieure de chacun des au moins deux réservoirs, pour favoriser la formation et le maintien d'une stratification en température.

Le système d'injection et de soutirage du fluide de stockage est agencé en partie inférieure pour prélever le fluide de stockage en partie inférieure de chacun des au moins deux réservoirs et injecter le fluide de stockage en partie inférieure de chacun des au moins deux réservoirs. L'unité de valorisation et le système d'injection et de soutirage est configuré pour favoriser la formation et le maintien d'une stratification en température préférentiellement lors d'une étape de charge des réservoirs, puis éventuellement lors d'une étape de décharge.

L'unité de valorisation comprend un module de gestion du module de stockage configuré pour contrôler le système d'injection ou de soutirage du fluide de stockage en fonction d'une consigne de température dans l'échangeur thermique.

Avantageusement, le circuit de stockage thermique est configuré pour fonctionner en circuit fermé.

Avantageusement, le recours à un module de stockage permet de récupérer et de stocker la chaleur fatale en vue de sa valorisation lors d'un cycle suivant.

A la différence des systèmes de l'état de la technique précédemment décrits qui proposent d'obtenir une stratification thermique par discrétisation physique du volume de stockage par niveau de température, la présente invention propose de maitriser une stratification thermique naturelle au sein d'un module de stockage thermique, en se basant sur l'effet physique « thermocline ».

La présente invention permet d'améliorer la qualité de l'énergie thermique valorisable, de simplifier la conception du ou des réservoirs et de maximiser sa compacité.

L'invention propose une unité de valorisation permettant de conserver l'intérêt de la stratification thermique naturelle tout en étant maitrisable et exploitable industriellement.

L'invention intègre dans le module de stockage un volume vide, qui sert de volume tampon.

De plus, le positionnement du système d'injection et de soutirage du fluide de stockage agencé en partie inférieure de chacun des au moins deux réservoirs va avoir pour conséquence de mélanger le fluide de stockage revenant de l'échangeur thermique lors de la phase de chauffage conduisant temporairement à l'homogénéisation de sa température dans un réservoir tout en permettant de stocker lors de la phase de refroidissement le fluide de stockage stratifié en température.

Un autre aspect concerne un dispositif de stérilisation comprenant un stérilisateur et une unité de valorisation telle que décrite ci-dessus. Le fluide de stockage est utilisé pour échanger de l'énergie thermique dans l'échangeur thermique avec un fluide de stérilisation configuré pour être utilisé dans le stérilisateur. Avantageusement, le dispositif de stérilisation comprend un stérilisateur associé à un échangeur thermique de l'unité de valorisation. Contrairement à l'état de la technique nécessitant l'utilisation de deux échangeurs thermiques pour un stérilisateur.

En acceptant de dégrader la qualité de l'énergie récupérée lors de la phase de chauffage (i.e. contenu dans le fluide de stockage revenant de l'échangeur), cette solution permet :
- d'être optimale pour la récupération de l'énergie thermique lors de la phase de refroidissement du stérilisateur, puis pour la valorisation de cette énergie thermique lors de la phase de chauffage du stérilisateur suivante.
- de gagner en simplicité pour la conception et la construction de la solution de stockage (gain sur la maturité et sur l'industrialisation à courts termes).

Un autre aspect concerne l'utilisation d'une unité de valorisation telle que décrite ci-dessus dans un dispositif de stérilisation.

Un autre aspect concerne un procédé de valorisation d'énergie thermique selon la revendication 9.

Les procédés et le dispositif selon la présente invention ont un fonctionnement particulier du fait de la récupération d'une chaleur fatale dont le niveau de température varie, en particulier diminue dans le temps. Plus spécifiquement, la chaleur fatale récupérée et stockée lors de la phase de refroidissement du stérilisateur diminue en température. L'injection en partie inférieure d'un réservoir préférentiellement du volume tampon conduit à ne pas altérer la qualité de l'énergie récupérée. Les procédés et dispositif selon la présente invention ont un fonctionnement particulier du fait que les besoins en température de la chaleur fatale stockée varie, en particulier augmente dans le temps. Lors de la phase de valorisation de l'énergie stockée, i.e. la décharge du système de stockage pour chauffer le stérilisateur, la température de l'eau doit augmenter progressivement pour suivre la consigne du barème de stérilisation. Les réservoirs de stockage précédemment chargés, doivent donc être déchargés en prélevant l'eau par le bas de sorte à progressivement extraire de l'eau de plus en plus chaude.

Les autres objets, caractéristiques et avantages de la présente invention apparaîtront à l'examen de la description suivante et des dessins d'accompagnement. Il est entendu que d'autres avantages peuvent être incorporés.

### BRÈVE DESCRIPTION DES FIGURES

Les buts, objets, ainsi que les caractéristiques et avantages de l'invention ressortiront mieux de la description détaillée d'un mode de réalisation de cette dernière qui est illustré par les dessins d'accompagnement suivants dans lesquels :
La figure 1 représente un schéma de tuyauterie et d'instrumentation (en anglais Piping and instrumentation diagram ou Process and instrumentation diagram, abrégé P&ID) simplifié d'un dispositif de stérilisation par fluide de stockage pressurisé surchauffé ruisselant, intégrant l'unité de valorisation selon l'invention.
La figure 2 représente un schéma de tuyauterie et d'instrumentation (P&ID) correspondant à la figure 1, le module de stockage 100 de l'unité de valorisation comprenant deux réservoirs.
La figure 3 représente un schéma de tuyauterie et d'instrumentation (P&ID) correspondant à la figure 1, le module de stockage 100 de l'unité de valorisation comprenant quatre réservoirs, l'état des réservoirs correspond à une fin de charge, à l'issue de la phase de refroidissement du stérilisateur 200, trois réservoirs sont remplis de fluide de stockage stratifié en température.
La figure 4 représente le même schéma de tuyauterie et d'instrumentation (P&ID) que la figure 3 - l'état des réservoirs correspond à une fin de décharge, au cours de la phase de chauffage du stérilisateur 200, trois réservoirs sont remplis de fluide de stockage à différents niveaux de température homogène.
La figure 5 représente le même schéma de tuyauterie et d'instrumentation (P&ID) que les figures 3 et 4 comprenant un système de pressurisation des réservoirs qui sont mis sous pression par un gaz.
La figure 6 représente un schéma de tuyauterie et d'instrumentation (P&ID) comprenant un système de pressurisation des réservoirs qui sont mis sous pression par un gaz et correspondant à un prototype du dispositif de stérilisation,
La figure 7 représente un même schéma de tuyauterie et d'instrumentation (P&ID) que la figure 6 à finalité industrielle.
La figure 8 représente un même schéma de tuyauterie et d'instrumentation (P&ID) que la figure 7 à finalité industrielle et comprenant une batterie de trois stérilisateurs et une unité de stérilisation mutualisées.

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques.

### DESCRIPTION DÉTAILLÉE

Avant d'entamer une revue détaillée de modes de réalisation de l'invention, sont énoncées ci-après des caractéristiques optionnelles qui peuvent éventuellement être utilisées en association ou alternativement.

Avantageusement, le volume tampon correspond au volume d'au moins un réservoir. Selon une possibilité, un réservoir ou réparti sur plusieurs réservoirs. Le volume tampon est avantageusement égal à un volume d'un réservoir. Selon une possibilité, le volume tampon correspond à un réservoir. Selon une autre possibilité, le volume tampon est égal ou inférieur au volume d'un réservoir et réparti sur les différents réservoirs de l'unité de valorisation.

Avantageusement, le circuit de stockage thermique 101 est configuré pour fonctionner en circuit fermé.

Avantageusement, le module de stockage 100 comprend un système d'homogénéisation et de répartition du débit du fluide de stockage injecté dans toute la section hydraulique des au moins deux réservoirs pour favoriser la formation et le maintien d'une stratification thermique du fluide de stockage chargé et déchargé dans les réservoirs.

Avantageusement, l'unité comprend un module de pressurisation 400 des au moins deux réservoirs de stockage configuré pour maintenir une pression suffisante pour le maintien du fluide de stockage à l'état liquide. Avantageusement, le module de pressurisation 400 comprend l'injection et l'évacuation d'un gaz à titre d'exemple de l'air ou l'azote. Avantageusement, le module de pressurisation 400 est agencé en partie supérieure des au moins deux réservoirs de stockage. Ce module de pressurisation 400 est particulièrement utile lorsque la température du fluide de stockage avoisine sa température d'évaporation.

Avantageusement, le module de stockage 100 comprend quatre réservoirs.

Avantageusement, l'unité de valorisation comprend un module de gestion du module de stockage 100 configuré pour contrôler l'injection ou le soutirage du fluide de stockage en fonction d'une consigne de température dans l'échangeur thermique.

Avantageusement, le circuit de stockage 101 comprend une première branche 104 agencée entre l'échangeur thermique 102 et le module de stockage 100 et configurée pour alimenter les au moins deux réservoirs de stockage et une deuxième branche 105 agencée entre le module de stockage 100 et l'échangeur thermique 102 configurée pour vider les au moins deux réservoirs de stockage.

Avantageusement, le dispositif de stérilisation comprend une arrivée de source froide différente du fluide de stockage et une arrivée de source chaude différente du fluide de stockage.

Avantageusement, au cours de l'étape de soutirage, le réservoir duquel est soutiré le fluide de stockage comprend le fluide de stockage à une température quasi homogène.

Avantageusement, au cours de l'étape d'injection, le volume tampon est un réservoir différent du réservoir de stockage duquel le fluide de stockage est soutiré, le fluide stockage est injecté en partie inférieure du réservoir et arrive de plus en plus froid assurant une stratification thermique du fluide de stockage dans le volume tampon.

Avantageusement, au cours de l'étape de soutirage, le réservoir duquel est soutiré le fluide de stockage comprend le fluide de stockage stratifié en température, le soutirage étant réalisé en partie inférieure, le prélèvement commence par le fluide de stockage le plus froid jusqu'au chaud, avantageusement situé à la surface libre,
Avantageusement, au cours de l'étape d'injection, le volume tampon est un réservoir différent du réservoir de stockage duquel le fluide de stockage est soutiré, le fluide stockage est injecté en partie inférieure du réservoir et arrive de plus en plus chaud et est mélangé avec le fluide de stockage contenu plus froid.

Il est précisé que dans le cadre de la présente invention, le terme « agencé sur », ou ses équivalents signifient « en connexion fluidique ».

Dans la présente description, l'expression « A fluidiquement raccordé à B» est synonyme de « A est en connexion fluidique avec B » ne signifie pas nécessairement qu'il n'existe pas d'organe entre A et B. Les expressions «agencée sur » ou «sur» sont synonymes de « raccordé fluidiquement à ».

L'amont et l'aval en un point donné sont pris en référence au sens de circulation du fluide dans le circuit.

L'unité de valorisation selon l'invention est avantageusement destinée à fonctionner avec au moins un stérilisateur 200 formant au moins en partie un dispositif de stérilisation.

Un dispositif de stérilisation est un dispositif destiné à stériliser des produits notamment par pulvérisation d'eau chaude ou de vapeur d'eau chaude.

Du point de vue énergétique, le fonctionnement des dispositifs de stérilisation est cyclique avec comme étapes successives : chauffe, maintien en température, puis refroidissement en cycle continu.

Classiquement, lors d'un procédé de stérilisation, la charge à l'intérieur de l'autoclave, aussi appelé stérilisateur 200, subit un cycle thermique assuré en chauffage par une source chaude telle qu'une chaudière et en refroidissement par une source froide telle que de l'eau de ville. La chaleur évacuée dans l'eau de ville lors des cycles de stérilisation représente un gisement énergétique conséquent qui peut s'élever à environ 300 kWh par cycle de stérilisation effectué avec un des modèles le plus vendu par STERIFLOW. Sachant que sur certains sites industriels, les stérilisateurs font jusqu'à 20 cycles par jour, les rejets sont supérieurs à 2 GWh et 50 000 m³ d'eau par an et par stérilisateur, si le refroidissement est assuré par de l'eau en circuit ouvert (= en eau perdue). La plupart du temps l'eau de refroidissement est en circuit fermé et est refroidie par une tour aéro-réfrigérante (TAR).

Le procédé de stérilisation est cadencé par lot, également dénommé par « batch » ne permettant pas l'utilisation d'un simple échangeur thermique 102 pour préchauffer l'eau entrant dans la chaudière. Le recours à l'utilisation d'un moyen de stockage pour récupérer et stocker la chaleur fatale en vue de sa valorisation lors d'un cycle suivant permet de s'affranchir du fait que les phases de chauffage et de refroidissement sont déphasées et interviennent à des moments différents.

Selon l'invention, l'unité de valorisation comprend un module de stockage thermique 100. Le module de stockage thermique 100 de l'unité de valorisation est avantageusement un stockage de chaleur sensible. L'unité de valorisation selon l'invention peut être installée sur des installations de stérilisation anciennes ou nouvelles.

Le module de stockage thermique 100 est apte à recevoir un fluide de stockage.

Le fluide de stockage est avantageusement un fluide caloporteur choisi pour fonctionner dans la température de l'application, en l'espèce pour un stérilisateur 200. À titre d'exemple, le fluide de stockage est de l'eau. Ce choix facilite la circulation successive du fluide de stockage, d'une source chaude et d'une source froide dans l'échangeur thermique 102 et notamment dans un premier circuit fluidique et un deuxième circuit fluidique de l'échangeur thermique. L'eau est préférentiellement pressurisée et potentiellement surchauffée. D'autres fluides peuvent être envisagés comme de l'huile thermique.

L'unité de valorisation selon l'invention comprend un circuit de stockage thermique 101. Le circuit de stockage thermique 101 est un circuit hydraulique avantageusement un circuit apte à fonctionner en circuit fermé. Le circuit de stockage 101 est raccordé au module de stockage 100 de sorte à stocker et déstocker le fluide de stockage. Le circuit de stockage 101 est apte à recevoir le fluide de stockage et avantageusement à assurer sa circulation.

L'unité de valorisation comprend avantageusement un échangeur thermique. Préférentiellement, l'unité de valorisation comprend un échangeur thermique 102 destiné à être associé à un stérilisateur 200. L'association se fait entre un échangeur thermique 102 et un stérilisateur 200. L'échangeur thermique 102 étant agencé sur le circuit de stockage thermique 101. Le fluide de stockage circulant dans le circuit de stockage 101 échange l'énergie thermique dans l'échangeur thermique 102 avantageusement avec un fluide de stérilisation destiné à circuler dans le stérilisateur 200 et à assurer la stérilisation. L'échangeur peut être par exemple un échangeur thermique de type tube /calandre ou bien à plaques.

L'unité de valorisation peut comprendre selon une possibilité plusieurs stérilisateurs. De la sorte l'unité de valorisation est mutualisée pour plusieurs échangeurs thermiques.

Le module de stockage thermique 100 comprend préférentiellement au moins deux réservoirs 110, 120, 130, 140. Dans la suite de la description, l'utilisation du terme réservoirs au pluriel se réfère à "au moins deux réservoirs". On entend par réservoir 110, 120, 130, 140 des volumes distincts dont l'injection et le soutirage de fluide de stockage est indépendant. Le module de stockage 100 est configuré pour permettre une stratification thermique naturelle. Avantageusement, les réservoirs 110, 120, 130, 140 s'étendent en hauteur de sorte à se conformer à des configurations favorables à l'établissement d'une stratification thermique dans leur volume.

Avantageusement, selon l'invention le fluide de stockage destiné à être stocké dans le module de stockage 100 représente un volume qui est inférieur au volume du module de stockage 100 de sorte à maintenir un volume vide dans le module de stockage 100. Ce volume tampon ne comprend pas du fluide de stockage. Ce volume vide est un volume tampon. Le volume tampon est présent avantageusement dans toutes les étapes d'utilisation de l'unité de valorisation, c'est à dire lors de la charge et de la décharge. Le volume tampon est destiné à permettre l'injection de fluide de stockage dans le volume tampon lors du soutirage du fluide de stockage dans un réservoir 110, 120, 130, 140 préférentiellement de sorte à ne pas mélanger le fluide de stockage destiné à être soutiré et le fluide de stockage injecté, c'est-à-dire le fluide de stockage destiné à l'échangeur thermique 102 et le fluide de stockage revenant de l'échangeur thermique. De manière temporaire, il est possible que le fluide de stockage soit temporairement soutiré puis injecté dans le même réservoir. Cette situation peut se produire par exemple lorsque la température du fluide déchargé de l'un des réservoirs de l'unité de valorisation ne permet pas de satisfaire la consigne sur la température (Tstock from limite) pour suivre le barème. En l'occurrence, cette situation apparait si lors de la charge du stockage, c'est-à-dire le refroidissement du stérilisateur, la température du réservoir débité est supérieure à la consigne ou si lors de la décharge du stockage, c'est-à-dire chauffage du stérilisateur, la température du réservoir débité est inférieure à la consigne, conduisant au changement de réservoir débité ou à l'arrêt de l'exploitation de la solution de stockage, laissant dans ce cas un réservoir partiellement vidé et un autre partiellement rempli.

La présence d'un volume tampon contribue à assurer la stratification thermique pour être à terme exploitée. Le volume tampon augmente l'énergie thermique pouvant être stockée dans le module de stockage 100 et facilite le stockage et déstockage du fluide de stockage.

Préférentiellement, le volume tampon correspond au volume d'au moins un réservoir 110, 120, 130, 140. Selon un mode de réalisation, le réservoir 110, 120, 130, 140 formant le volume tampon change en fonction des phases de stockage et déstockage du fluide de stockage, différents réservoirs 110, 120, 130, 140 sont utilisés alternativement comme volume tampon. Selon une possibilité, chaque réservoir 110, 120, 130, 140 est alternativement utilisé comme un volume tampon.

Selon un mode de réalisation, les réservoirs 110, 120, 130, 140 sont utilisés en vase communicant.

Dans une configuration plus avantageuse en matière de densité énergétique de stockage et de qualité de l'énergie thermique stockée, le module de stockage 100 est avantageusement composé de plus de deux réservoirs 110, 120, 130, 140. En effet suivant la présente invention, plus le nombre de réservoirs 110, 120, 130, 140 est important, plus le volume (vide) tampon est faible comparativement au volume de stockage valorisé énergétiquement. À titre d'exemple si le module de stockage 100 comprend deux réservoirs 110, 120, 130, 140, seulement la moitié du volume total de stockage est utilisé alors que si le module de stockage 100 compte quatre réservoirs, les trois quarts du volume total de stockage sont utilisés. Plus le nombre de réservoir est important, plus le ratio du nombre de réservoir vide sur le nombre de réservoir total est faible.

De même, plus le nombre de réservoirs 110, 120, 130, 140 est important, plus il sera possible de constituer une stratification thermique en discrétisant par différents niveaux de température homogènes, les réservoirs de stockage remplis lors de la phase de décharge c'est à dire lors du chauffage du stérilisateur 200 ; phase durant laquelle une stratification thermique naturelle est difficile à maitriser, sans modifier la conception du module de stockage. Cette configuration est donc particulièrement avantageuse énergétiquement puisqu'en fin de phase de chauffage du stérilisateur 200, les réservoirs 110, 120, 130, 140 sont remplis avec un fluide de stockage à différents niveaux de température (homogène).

Le module de stockage 100 comprend avantageusement un système d'injection et de soutirage du fluide de stockage 108 des réservoirs 110, 120, 130, 140. Préférentiellement, le système d'injection et de soutirage 108 est agencé en partie inférieure de chacun des réservoirs 110, 120, 130, 140. Préférentiellement, le module de stockage 100 comprend un système d'injection et de soutirage 108 par réservoir. L'agencement du système d'injection et de soutirage 108 en partie inférieure des réservoirs 110, 120, 130, 140 assure une simplicité de fabrication et d'utilisation du module de stockage 100. Cet emplacement conduit à mélanger le fluide de stockage injecté dans au moins un des réservoirs 110, 120, 130, 140 lors d'une étape de décharge du module de stockage 100. Durant cette étape de décharge, un débit de fluide de stockage revenant de l'échangeur thermique 102 est injecté dans réservoir par la partie inférieure dans un volume de fluide de stockage déjà présente qui est à une température plus faible, entrainant un mélange et donc une homogénéisation de leur température. Ce mélange conduit à une homogénéisation temporaire de la température du fluide de stockage au sein du réservoir. Les effets néfastes potentiels sur le rendement du module de stockage sont avantageusement compensés par la présence du volume tampon. Lors du soutirage du fluide de stockage depuis un réservoir pour assurer le chauffage du stérilisateur 200, le fluide de stockage est initialement stratifié en température (puisqu'il a été stratifié lors de la précédente phase de refroidissement du stérilisateur).

L'homogénéisation de la température est une conséquence inhérente à l'injection par la partie inférieure des réservoirs d'un débit de fluide de stockage plus chaud (revenant de l'échangeur) dans un volume de fluide de stockage plus froid déjà présent dans le réservoir du module de stockage uniquement lors de la phase de chauffage.

Le soutirage du fluide de stockage quasi homogène en température ne se produit donc que lors de la phase de refroidissement du stérilisateur200.

Ensuite, toujours lors de la phase de refroidissement du stérilisateur 200, le fluide de stockage revenant de l'échangeur 102 est par contre injecté dans le stockage en maitrisant une stratification thermique. Cette stratification thermique est rendue possible par le fait que le fluide de stockage revenant de l'échangeur thermique 102 lors de la phase de refroidissement est de plus en plus froid et est injecté par la partie inférieure du réservoir.

Selon un mode de réalisation, le module de stockage 100 comprend un système d'homogénéisation et de répartition du débit du fluide de stockage injecté dans toute la section hydraulique des réservoirs 110, 120, 130, 140. À titre d'exemple, un système d'homogénéisation et de répartition du débit du fluide de stockage peut comprendre des dispositifs avantageusement dimensionnés et conçus pour répartir uniformément le débit du fluide de stockage injecté et soutiré dans le réservoir sur une plus grande section hydraulique de façon à réduire sa vitesse d'écoulement dans celui-ci. En effet, plus la vitesse d'écoulement est importante, plus le brassage hydraulique qu'elle engendrera augmentera, ce qui est très préjudiciable à la formation et au maintien d'une stratification thermique au sein du volume de stockage. Ces dispositifs sont par exemple des déflecteurs hydrauliques dont la forme peut être élaborée afin d'assurer une bonne orientation et une bonne distribution du débit dans le réservoir tout en réduisant sa vitesse d'écoulement.

Le circuit de stockage 101 comprend une première branche 104 provenant de l'échangeur thermique 102 et alimentant le module de stockage thermique 100 (stock-to). La première branche 104 est connectée fluidiquement à une sortie de l'échangeur thermique 102 et à une entrée du module de stockage 100. Le circuit de stockage 101 comprend d'une deuxième branche 105 provenant du module de stockage thermique 100 et alimentant l'échangeur thermique 102 (stock-from). La deuxième branche 105 est connectée fluidiquement à une sortie du module de stockage 100 et à une entrée de l'échangeur thermique.

Le circuit de stockage 101 est configuré pour fonctionner en circuit fermé.

Le module de stockage 100 et en particulier les réservoirs 110, 120, 130, 140 sont raccordés au circuit de stockage 101 en parallèle. Chaque réservoir 110, 120, 130, 140 sont raccordés à la première branche 104 et à la deuxième branche 105 respectivement par une première conduite 11 et une deuxième conduite 12. La première conduite 11 est raccordée fluidiquement à la première branche 104 assurant l'alimentation du réservoir en fluide de stockage. La deuxième conduite 12 est raccordée fluidiquement à la deuxième branche 105 assurant la sortie du fluide de stockage du réservoir.

Chaque conduite 11, 12 comprend avantageusement une vanne d'isolement hydraulique thermiquement commandée. L'ouverture ou la fermeture de la vanne d'isolement est contrôlée en fonction de la température du fluide de stockage, de préférence mesurée au niveau de son point de prélèvement, et d'une consigne de température prédéfinie, préférentiellement évaluée en fonction de la température à atteindre par le fluide de stérilisation à sa sortie de l'échangeur thermique (T_{STW,to}) lorsqu'il est chauffé ou refroidit respectivement par une source chaude ou par une source froide, pouvant être le fluide de stockage . Les vannes d'isolement sont dénommées V_{stack - from} pour les vannes de contrôle de la sortie du fluide de stockage d'un réservoir. Elles sont avantageusement agencées sur les deuxièmes conduites 12. Les vannes d'isolement sont dénommées V_{stock - to} pour les vannes de contrôle de l'entrée du fluide de stockage dans un réservoir 110, 120, 130, 140. Elles sont avantageusement agencées sur les premières conduites 11. Ces vannes d'isolement V_{stock - from} et/ou V_{stack - to} permettent ainsi de sélectionner le réservoir qui doit être déchargé et le réservoir 110, 120, 130, 140 qui doit être chargé, notamment en fonction de leurs états et de la phase du cycle de stérilisation opéré. Avantageusement, ces vannes d'isolement V_{stock - from} et/ou V_{stack - to} sont des vannes tout ou rien, c'est-à-dire ouvert/fermé.

L'unité de valorisation selon l'invention comprend avantageusement une vanne VSV-Q agencée sur le circuit de stockage préférentiellement en aval du débitmètre 109. La vanne VSV-Q est destinée à assurer la variation du débit du fluide de stockage dans le circuit de stockage.

Suivant un mode de réalisation privilégié de cette unité de valorisation permettant une solution de stockage thermique à l'échelle industrielle, une seule et même vanne de régulation de débit VSV-Q peut être utilisée à la fois pour le circuit de stockage et pour l'eau de refroidissement. Il s'agit ainsi d'une seule et même vanne assurant la régulation du débit de fluide caloporteur circulant dans l'échangeur thermique 102 du stérilisateur 200 et provenant soit du circuit de stockage, soit d'une arrivée d'eau de refroidissement 302. Cette double utilisation de la vanne de régulation de débit VSV-Q est possible car le circuit de stockage et l'arrivée d'eau de refroidissement 302 sont découplés et ne sont pas amenés à travailler simultanément sur le même stérilisateur 200. L'avantage inhérent à cette mutualisation de la vanne de régulation de débit VSV-Q, est l'économie d'achat d'une vanne de régulation supplémentaire (coûteuse) et donc la réduction du coût d'investissement de la solution de stockage thermique.

De la même manière, le débitmètre 109 peut être à la fois utilisé pour mesurer le débit d'eau alimentant l'échangeur thermique 102 en provenance soit du circuit de stockage soit de l'arrivée d'eau de refroidissement 302. Selon cette possibilité, le circuit de stockage comprend en aval de la pompe 107 le débitmètre 109, puis une connexion d'une branche d'arrivée d'eau de refroidissement 302 assurant l'arrivée d'eau de refroidissement 302 puis la vanne de régulation VSV-Q.

L'unité de valorisation selon l'invention comprend avantageusement une instrumentation thermique comprenant des thermomètres destinés à mesurer la température du fluide de stockage à différents points de l'unité de valorisation. Selon une préférence, l'instrumentation thermique comprend également des thermomètres destinés à mesurer la température du fluide de stérilisation entrant et sortant de l'échangeur thermique 102 respectivement revenant ou en direction du stérilisateur 200. Sur les figures, les thermomètres de l'instrumentation thermique sont dénommés soit T_{stock} pour les thermomètres concernant le fluide de stockage soit T_{STW} pour les thermomètres concernant le fluide de stérilisation.

Avantageusement, pour garantir son autonomie, le circuit de stockage thermique 101 est pourvu de sa propre pompe 107 assurant la circulation du fluide de stockage. Avantageusement, l'unité de valorisation peut se satisfaire d'une seule pompe 107 et même dans le cas d'une mutualisation de l'unité de valorisation à plusieurs stérilisateurs. En effet, le circuit de stockage 101 fonctionnant en circuit fermé, tout le débit prélevé est réinjecté.

Selon une possibilité, pour maintenir le fonctionnement de la pompe 107 à son régime nominal, malgré les variations du débit de fluide de stockage, le circuit de stockage comprend une branche de dérivation 106 assurant un routage en by-pass du fluide de stockage. Avantageusement, la circulation du fluide de stockage dans la branche de dérivation 106 est régulée par la vanne V_{by} encadrant la pompe 107 préférentiellement au niveau de ses bornes. Selon une autre possibilité, la pompe 107 peut être équipée d'un variateur de fréquence sur lequel il est possible d'agir de façon à piloter le débit de fluide de stockage circulant dans le circuit de stockage et ce en faisant varier la vitesse de rotation de la pompe.

Compte tenu de la température potentiellement élevée du fluide de stockage chargé dans les réservoirs 110, 120, 130, 140 pouvant s'approcher de la température d'évaporation du fluide de stockage, tel que 100°C pour de l'eau à la pression atmosphérique, selon un mode de réalisation de l'invention, l'unité de valorisation comprend avantageusement un système de pressurisation 400 du module de stockage 100. Le module de pressurisation 400 est configuré pour mettre sous pression les réservoirs 110, 120, 130, 140 de façon à maintenir à l'état liquide le fluide de stockage et notamment de l'eau surchauffée.

Pour solutionner la contrainte du niveau variable des réservoirs110, 120, 130, 140 qui pourrait conduire à dépressuriser le réservoir lors de la phase de décharge de celui-ci par soutirage, le module de pressurisation 400 est configuré pour pressuriser la surface libre en utilisant par exemple un gaz tel que l'air ou l'azote. Le module de pressurisation 400 est avantageusement mis en place et connecté à la partie supérieure de chaque réservoir 110, 120, 130, 140 pour maintenir une pression suffisante indispensable au maintien du fluide de stockage et notamment de l'eau surchauffée à l'état liquide.

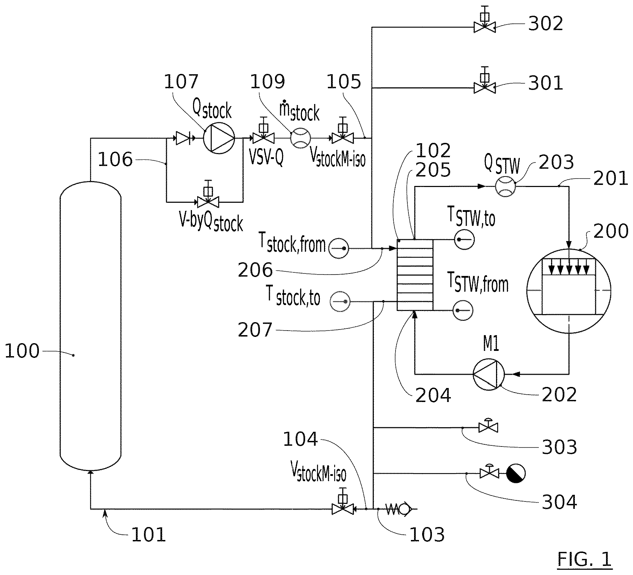

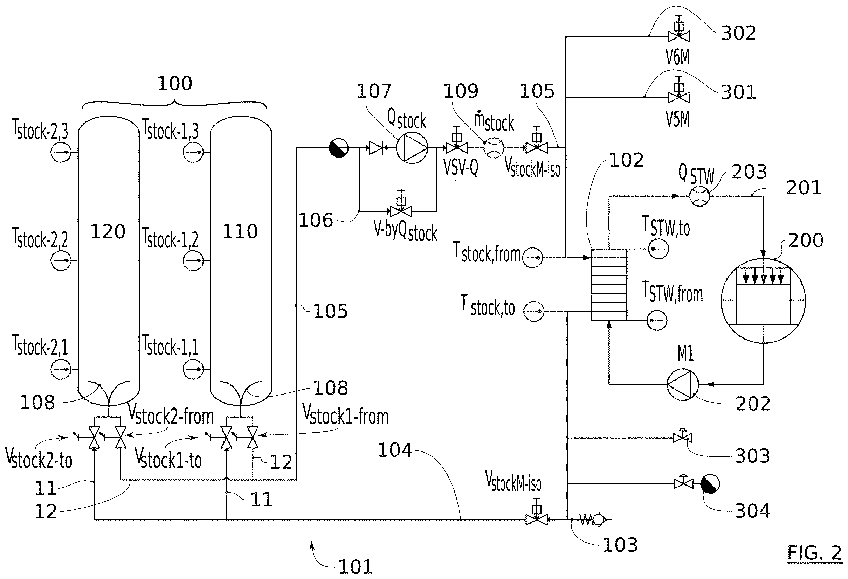

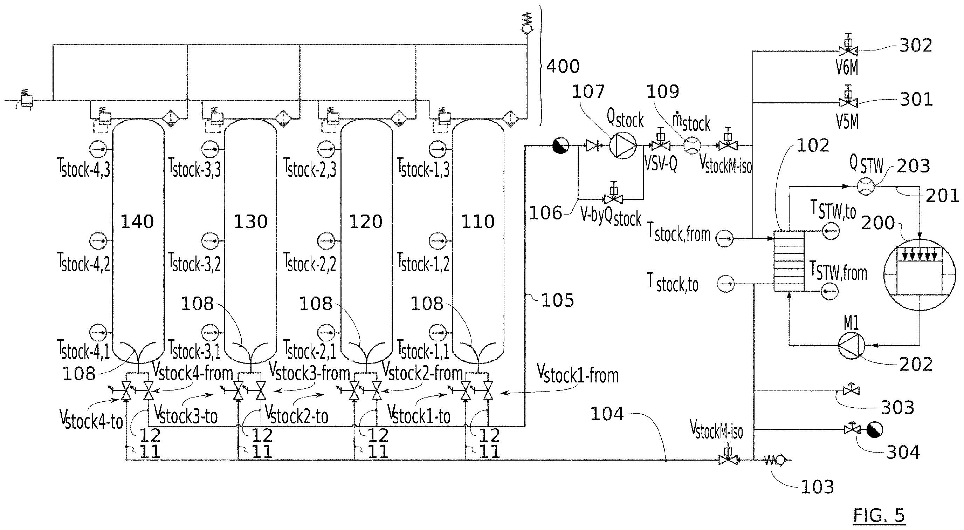

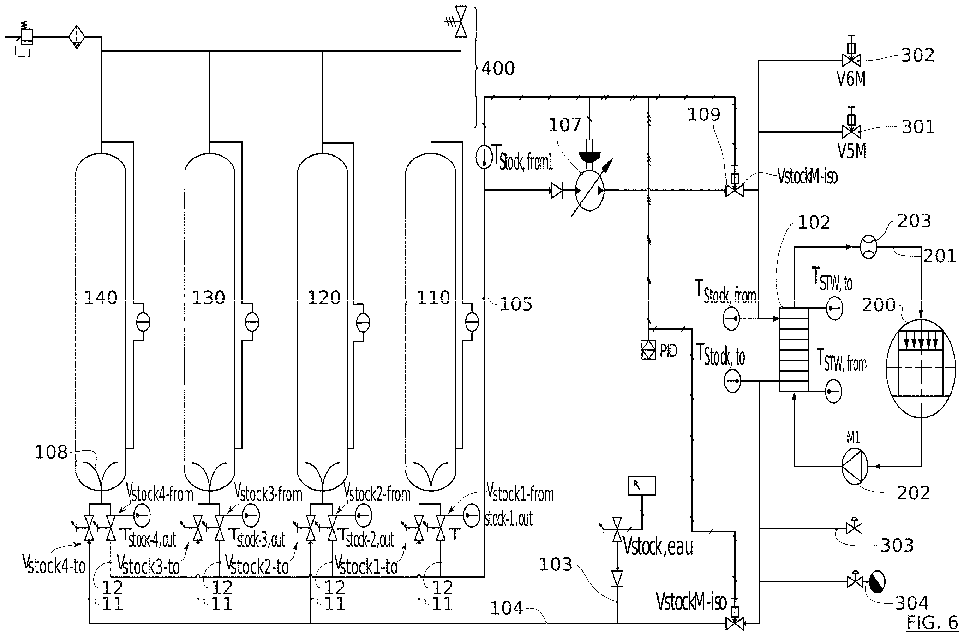

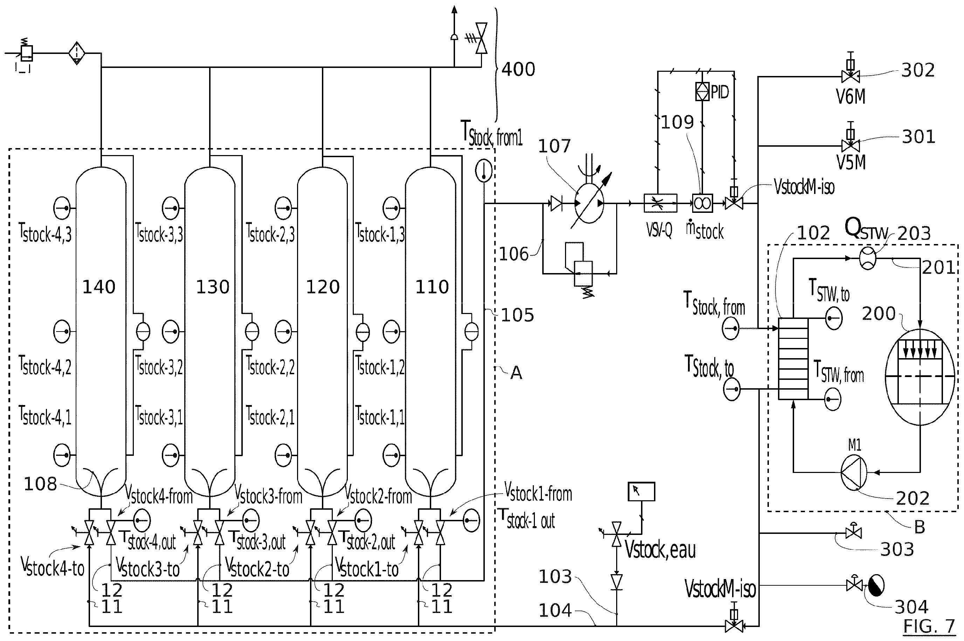

Le module de pressurisation 400 est configuré pour fonctionner préférentiellement en vase communiquant. A cet effet, le module de pressurisation 400 comprend un circuit de gaz reliant tous les réservoirs 110, 120, 130, 140 préférentiellement par leur partie supérieure de façon à maintenir une pression homogène dans chacun d'eux, quel que soit leur état de remplissage en fluide de stockage. Pour limiter l'ingestion de fluide de stockage d'un réservoir à l'autre par l'intermédiaire de ce circuit, le module de pressurisation 400 comprend avantageusement un purgeur automatique placé entre le sommet de chaque réservoir et le circuit de gaz pressurisant. Un exemple est illustré en figure 5. Un autre exemple de système de pressurisation 400 est illustré en figure 6. Le système de pressurisation 400 de la figure 6 est plus simplifié que celui de la figure 5. En figure 7, le système de pressurisation 400 illustré ne présente pas de disque de rupture qui est un organe de sécurité supplémentaire agencé sur le système de pressurisation. Le système de pressurisation 400 notamment celui illustré en figure 6 est configuré pour être agencé sur les unités de valorisations illustrées aux figures 1 à 4.

Selon un mode de réalisation, l'unité de valorisation selon l'invention comprend un débitmètre massique 109 (ṁ_{stock}) agencé sur le circuit de stockage 101, plus préférentiellement sur la deuxième branche 105 du circuit et plus préférentiellement en aval de la pompe 107. Le débitmètre 109 assure la mesure du débit de fluide de stockage circulant dans le circuit de stockage 101 et destiné à alimenter l'échangeur thermique 102. Dans ce cas-là, le débit du fluide de stockage est avantageusement régulé en fonction des mesures de température aux bornes l'échangeur thermique 102 de façon à ce que la température de consigne du fluide de stérilisation soit atteinte à la sortie de l'échangeur thermique 102 (T_{stw, to}). Selon un autre mode de réalisation, le pilotage du débit du fluide de stockage s'affranchit de l'utilisation d'un débitmètre. Ce mode de pilotage repose sur l'évaluation de l'efficacité de l'échangeur thermique 102 en se basant sur les mesures de température à ses bornes et sur la température de consigne que le fluide de stérilisation doit atteindre à la sortie de l'échangeur thermique (T_{STW, to}). Une corrélation de l'efficacité de l'échangeur thermique 102 en fonction du débit du fluide de stockage a pu être mise au point. Cette relation qui permet de corréler l'efficacité de l'échangeur thermique 102 en fonction du débit du fluide de stockage repose sur le fait que le débit très élevé et constant (à titre d'exemple 265 m3/h) du fluide de stérilisation dans le circuit de stérilisation 201 implique que la résistance thermique limitante se situe toujours du côté du circuit de stockage 101, quand celui-ci est utilisé. Il est donc possible de piloter le débit du fluide de stockage et donc la puissance thermique transférée, en évaluant l'efficacité de l'échangeur thermique 102 à partir des températures mesurées à ses bornes et de la température de consigne que le fluide de stérilisation doit atteindre à la sortie de l'échangeur thermique (T_{STW, to}).

Selon une possibilité avantageuse, l'unité de valorisation comprend au moins deux vannes d'isolement V_{stock M-ISO} du circuit de stockage 101 par rapport à l'échangeur thermique. Les vannes d'isolement V_{stock M-Iso} sont agencées sur le circuit de stockage 101 avantageusement une sur la première branche 104 et l'autre sur la deuxième branche 105. Préférentiellement, les vannes d'isolement V_{stock M-ISO} sont agencées aux bornes de l'échangeur thermique. Ces vannes sont configurées pour assurer l'isolement du circuit de stockage 101 et donc arrêter la circulation du fluide de stockage dans l'échangeur thermique 102 en particulier dans l'objectif de permettre la circulation dans l'échangeur thermique 102 d'une source froide ou d'une source chaude venant en relai du fluide de stockage. Avantageusement, ces vannes V_{stack M-ISO} sont des vannes tout ou rien, ouvert / fermé.

De manière préférée, les vannes d'isolement V_{stock M-ISO} sont robotisées et pilotées par un module de gestion.

Selon un mode de réalisation, l'unité de valorisation comprend un module de gestion de l'énergie thermique. Le module de gestion est configuré pour assurer la valorisation optimale de l'énergie thermique destiné à au moins un stérilisateur 200. Le module de gestion pilote préférentiellement les vannes d'isolement des réservoirs V_{stock-from} et V_{stock-to} ainsi que les vannes d'isolement du circuit de stockage 101 V_{stock M-Iso}. Selon une possibilité, le module de gestion évalue l'efficacité de l'échangeur thermique 102 à partir des températures mesurées à ses bornes et de la température de consigne que le fluide de stérilisation doit atteindre à la sortie de l'échangeur thermique (T_{STW, to}). Dans ce cas-là, le module de gestion pilote avantageusement une pompe 107 équipée d'un variateur de fréquence sur lequel il est possible d'agir de façon à piloter le débit de fluide de stockage circulant dans le circuit de stockage et ce en faisant varier la vitesse de rotation de la pompe. Selon une autre possibilité, le module de gestion pilote la vanne VSV-Q destiné à module le débit du fluide de stockage en entrée du stérilisateur 200. Le module de gestion collecte des informations de l'unité de valorisation par diverses instrumentations que ce soit le débit du fluide de stockage circulant par le débitmètre 109, et/ou les températures du fluide de stockage et/ou du fluide de stérilisation par l'instrumentation thermique décrite ci-dessus.

Le module de gestion est destiné à satisfaire une consigne de température pour assurer un fonctionnement correct du stérilisateur 200. Une température de consigne est prédéfinie. Préférentiellement, cette température de consigne est celle du fluide de stérilisation en sortie de l'échangeur thermique 102 (T_{STW,to}). Selon une possibilité, cette consigne de température varie en fonction de la phase de fonctionnement du stérilisateur 200. La consigne de température suit un barème qui correspond au fonctionnement du stérilisateur 200.

Le module de gestion pilote ainsi le débit de fluide de stockage (ṁ_{stock}) circulant avantageusement en boucle fermée dans le circuit de stockage 101 en modulant la vanne de régulation VSV-Q.

Avantageusement, le module de gestion assure le pilotage des vannes d'isolement des réservoirs en fonction de la température du fluide de stockage contenue dans lesdits réservoirs et des besoins en énergie thermique du stérilisateur 200. Le module de gestion pilote l'ordre du stockage et du déstockage du fluide de stockage des réservoirs.

Le dispositif de stérilisation selon l'invention illustré aux différentes figures 1 à 7 comprend une partie commune reproduite sur la droite de l'ensemble des figures et décrite ci-dessous. Le dispositif de stérilisation comprend un stérilisateur 200 alimenté par un fluide de stérilisation circulant avantageusement en boucle fermée dans un circuit de stérilisation 201 entre l'échangeur thermique 102 et le stérilisateur 200. Le circuit de stérilisation 201 comprend avantageusement une pompe 202 et éventuellement un débitmètre massique 203. L'échangeur thermique 102 comprend une entrée 204 et une sortie 205 du fluide de stérilisation et une entrée 206 et une sortie 207 d'une source chaude ou froide selon les besoins. Selon une possibilité avantageuse, la source chaude est soit de la vapeur provenant d'un moyen de chauffage tel que par exemple une chaudière, soit un fluide de stockage. De même la source froide est soit de l'eau de refroidissement provenant d'un réseau d'eau, soit le fluide de stockage.

Sur les figures sont donc illustrées une arrivée d'eau de refroidissement 302, une arrivée de vapeur 301 connectées à l'entrée 206 de l'échangeur thermique 102 et une sortie d'eau de refroidissement 303 et une sortie de condensat 304 connectées à la sortie 207 de l'échangeur thermique 102.

Une telle unité de valorisation correctement dimensionnée, conçue et pilotée permettrait de valoriser en régime stabilisé de l'ordre de 30% de l'énergie thermique nécessaire pour assurer la phase de chauffage et l'énergie thermique nécessaire pour dissiper la chaleur lors de la phase de refroidissement du procédé de stérilisation, en récupérant et en stockant la chaleur fatale extraite du stérilisateur 200 lors de la phase de refroidissement du cycle précédent. Une telle solution pourrait trouver une rentabilité sur des très courtes durées par exemple inférieures à 3-4 ans, particulièrement si dans ce contexte le procédé de stérilisation auquel elle est couplée, exécute un grand nombre de cycles par jour, par exemple supérieur à 5 cycles par jour.

L'unité de valorisation selon l'invention permet de valoriser une quantité d'énergie thermique entre les cycles de stérilisation en réduisant ainsi les quantités de vapeur et d'eau de refroidissement consommées, mais contribue également à augmenter la disponibilité des utilités (vapeur, eau de refroidissement). En effet, lorsque les autoclaves ou stérilisateurs sont chauffés ou sont refroidis grâce à l'unité de valorisation, ils ne soutirent pas de débit sur les réseaux de vapeur ou d'eau de refroidissement, augmentant par voie de conséquence leur capacité et leur disponibilité pour les autres autoclaves (stérilisateurs ou équipements) n'étant pas alimentés par l'unité de valorisation. Cet avantage sera particulièrement intéressant sur les installations qui rencontrent des problèmes de congestion des utilités, conduisant à limiter soit le nombre d'appareils pouvant être alimentés en parallèle, soit la vitesse des montées et des descentes en température (rampes - suivi du barème). En outre, dans le cas d'installation d'autoclaves - stérilisateurs supplémentaires sur des réseaux déjà existants, l'intégration d'unité de valorisation pourrait permettre possiblement de limiter, voire d'éviter l'investissement dans des moyens de chauffage et/ou de refroidissement d'appoint pour assurer leurs alimentations.

L'invention concerne un procédé de valorisation d'énergie thermique par une unité de valorisation telle que décrite ci-dessus. Le procédé de valorisation comprend avantageusement une étape de décharge alternativement avec une étape de charge.

La charge du module de stockage 100 correspond au stockage du fluide de stockage ayant récupéré de l'énergie thermique au niveau de l'échangeur thermique 102. Cette étape de charge du module de stockage 100 correspond à la phase de refroidissement du stérilisateur 200. Au cours de cette phase de refroidissement, le stérilisateur 200 et son chargement sont refroidis par circulation du fluide de stérilisation refroidi au niveau de l'échangeur thermique 102 par échange thermique avec une source froide pouvant être de l'eau de refroidissement ou avec le fluide de stockage. Dans le cas d'une étape de charge du module de stockage 100, c'est bien le fluide de stockage qui circule dans l'échangeur thermique 102 pour échanger avec le fluide de stérilisation pour assurer son refroidissement.

En début d'étape de charge, c'est-à-dire en fin d'étape de décharge, le module de stockage 100 contient du fluide de stockage à l'exception du volume tampon, correspondant avantageusement à un réservoir 110, 120, 130, 140 maintenu vide. Le ou les réservoir(s) contenant du fluide de stockage est (sont) entièrement ou partiellement rempli(s). Préférentiellement, dans le cas où le module de stockage 100 comprend plus de deux réservoirs 110, 120, 130, 140, leur niveau est identique du fait du fonctionnement en vase communicant. L'optimum de valorisation énergétique implique pas forcément de remplir au volume maximal les réservoirs à l'exception du volume tampon. Ceci est particulièrement vrai au démarrage quand le fluide de stockage contenu initialement dans les réservoirs est proche de la température ambiante. Le fluide de stockage contenu dans chaque réservoir 110, 120, 130, 140 est avantageusement à une température quasi homogène dans tout le volume du réservoir 110, 120, 130, 140.

L'étape de charge comprend le soutirage du fluide de stockage d'un réservoir 110, 120, 130, 140 de stockage, tel que par exemple le premier réservoir 110 de stockage. Le fluide de stockage est prélevé en partie inférieure par le système d'injection et de soutirage du fluide de stockage 108. Le fluide de stockage est récupéré par le circuit de stockage 101, plus précisément la deuxième branche 105, assurant la circulation du fluide de stockage en direction de l'échangeur thermique 102. Le prélèvement commence par le réservoir 110, 120, 130, 140 dont le fluide de stockage présente la température la plus chaude. Le prélèvement se poursuit par la température décroissante du fluide de stockage; du plus chaud jusqu'au froid.

L'étape de charge comprend avantageusement simultanément l'injection du fluide de stockage dans un réservoir de stockage 110, 120, 130, 140, préférentiellement différent du réservoir de stockage 110, 120, 130, 140 duquel le fluide de stockage est soutiré, tel que par exemple le deuxième réservoir de stockage 120. Le fluide de stockage est injecté dans le volume tampon. Préférentiellement, le réservoir 110, 120, 130, 140 destiné à recevoir le fluide de stockage correspond au réservoir formant le volume tampon. Le réservoir 110, 120, 130, 140 destiné à recevoir le fluide de stockage est avantageusement vide. Le fluide de stockage arrive de plus en plus froid, permettant de maitriser une stratification thermique en conservant en partie supérieure le fluide de stockage le plus chaud issu du début du refroidissement du stérilisateur 200 et en partie basse le fluide de stockage le plus froid injecté.

La décharge du module de stockage 100 correspond au déstockage du fluide de stockage transférant son énergie thermique au niveau de l'échangeur thermique 102. Cette étape de décharge du module de stockage 100 correspond à la phase de chauffage du stérilisateur 200. Au cours de cette phase de chauffage, le stérilisateur 200 et son chargement sont chauffés par circulation du fluide de stérilisation chauffé au niveau de l'échangeur thermique 102 par échange thermique avec une source chaude pouvant être de la vapeur d'eau ou avec le fluide de stockage. Dans le cas d'une étape de décharge du module de stockage 100, c'est bien le fluide de stockage qui circule dans l'échangeur thermique 102 pour échanger avec le fluide de stérilisation pour assurer son chauffage.

En début d'étape de décharge, c'est à dire en fin d'étape de charge, les réservoirs contiennent du fluide de stockage à l'exception du volume tampon correspondant avantageusement à un réservoir 110, 120, 130, 140 maintenu vide. Les réservoirs 110, 120, 130, 140 contenant du fluide de stockage sont entièrement ou partiellement remplis. Préférentiellement, dans le cas où le module de stockage 100 comprend plus de deux réservoirs 110, 120, 130, 140, leur niveau est identique, du fait du fonctionnement en vase communicant. Le fluide de stockage contenu dans chaque réservoir est avantageusement stratifié en température.

La décharge du module de stockage 100 s'effectue avantageusement suivant un mode opératoire qui permet de limiter l'altération de la stratification thermique établie dans les réservoirs 110, 120, 130, 140 précédemment chargés de sorte à ne pas dégrader l'énergie thermique contenue dans chacun d'eux. A l'inverse de la charge, lors de la décharge les réservoirs 110, 120, 130, 140 remplis composant le module de stockage 100 sont débités pour alimenter l'échangeur thermique 102 couplé au stérilisateur 200, en commençant par celui qui contient du fluide de stockage à plus basse température puis en enchaînant ensuite par ordre croissant de température jusqu'au réservoir 110, 120, 130, 140 stockant du fluide de stockage à la température la plus élevée. Avantageusement, les réservoirs 110, 120, 130, 140 contenant un fluide de stockage stratifié à plus haute température sont réservés pour la fin de la décharge au moment où le chauffage du stérilisateur 200 requiert des températures de fluide de stérilisation de plus en plus élevées.

L'étape de décharge comprend le soutirage du fluide de stockage d'un réservoir de stockage110, 120, 130, 140, tel que par exemple le deuxième réservoir 120 de stockage. Le fluide de stockage est prélevé en partie inférieure par le système d'injection et de soutirage du fluide de stockage 108. Le fluide de stockage du réservoir 110, 120, 130, 140 est stratifié en température. Le fluide de stockage est récupéré par le circuit de stockage 101, plus précisément par la deuxième branche 105 en direction de l'échangeur thermique 102. Lors de l'étape de décharge, dans le cas où plusieurs réservoirs sont remplis de fluide de stockage, le réservoir 110, 120, 130, 140 déstocké en premier est celui dont la température du fluide de stockage est la plus basse. Le soutirage s'effectue successivement dans les réservoirs par ordre croissant de température. Le fluide de stockage présentant la haute température est réservé à la fin de la décharge au moment où le chauffage du stérilisateur 200 requière des températures de fluide de stérilisation de plus en plus élevées.

L'étape de décharge comprend avantageusement simultanément l'injection du fluide de stockage dans un réservoir de stockage 110, 120, 130, 140 préférentiellement différent du réservoir de stockage duquel le fluide de stockage est soutiré, tel que par exemple le premier réservoir de stockage 110. Le fluide de stockage est injecté dans le volume tampon. Préférentiellement, le réservoir 110, 120, 130, 140 destiné à recevoir le fluide de stockage correspond au réservoir 110, 120, 130, 140 formant le volume tampon. Le réservoir destiné à recevoir le fluide de stockage est avantageusement vide. Le fluide de stockage est avantageusement injecté par la partie inférieure. Le fluide de stockage injecté par la partie inférieure arrive de plus en plus chaude et est mélangé avec le fluide de stockage contenu plus froid. L'injection en partie inférieure du fluide de stockage conduit à l'homogénéisation de sa température puisque l'injection en partie inférieure d'un débit de fluide de stockage plus chaud dans un volume de fluide de stockage plus froid entrainera naturellement des mouvements de convection naturelle, induit par la différence de densité des masses de fluide de stockage à températures différentes, engendrant un brassage qui conduira à une homogénéisation de la température du fluide de stockage stocké. Avantageusement, dans la mesure où suivant cette conception l'injection et le soutirage du débit de fluide de stockage s'effectuent toujours en partie inférieure du réservoir, l'homogénéisation de sa température à l'intérieur des réservoirs au cours de la phase de chauffage du stérilisateur constitue un cas favorable pour la phase de refroidissement suivante puisqu'il permettra d'éviter le soutirage d'un débit d'eau prélevé au sein d'un même réservoir, de plus en plus chaud à température stratifiée qui serait antagoniste avec le besoin de refroidissement du stérilisateur.

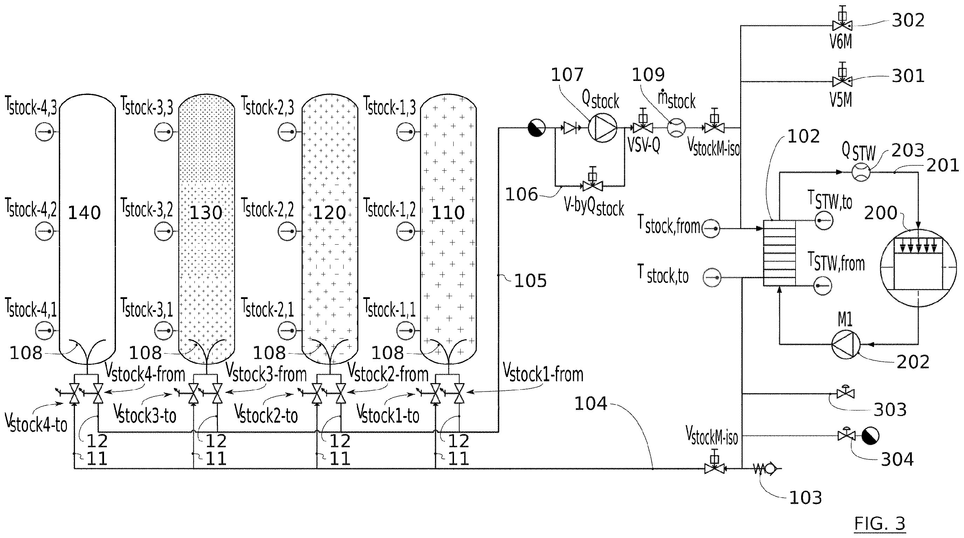

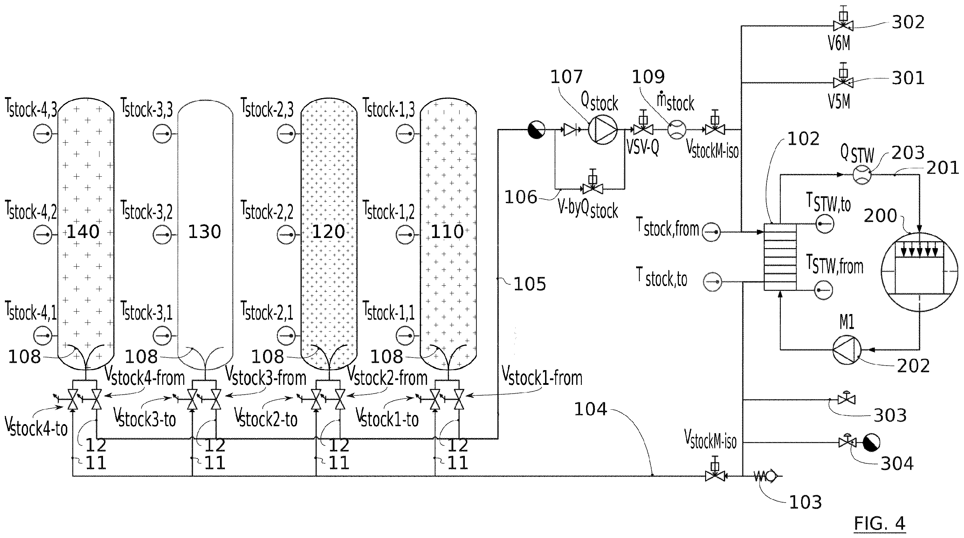

Les figures 3 et 4 sont décrites ci-après. Elles illustrent l'état du module de stockage 100 composé de quatre réservoirs 110, 120, 130, 140 à deux moments caractéristiques de leur exploitation, à savoir : la fin de leur charge, figure 3 et la fin de leur décharge, figure 4.

Exemple de charge du module de stockage 100 de l'unité de valorisation:
L'étape de charge débute à la suite d'une étape de décharge. Les réservoirs 110, 120, 130, 140 sont dans un état initial correspondant à la figure 4.

L'étape de charge du module stockage s'effectue pendant la phase de refroidissement du stérilisateur. Tel qu'illustré sur la figure 4, au début de l'étape de charge du stockage 100, les trois réservoirs 110, 120 et 140 contiennent du fluide de stockage chacun à une température homogène différente. Le réservoir 120 contient du fluide de stockage à la température homogène la plus élevée (par rapport aux autres réservoirs), tandis que le réservoir 140 contient du fluide de stockage à la température homogène la plus faible. Le réservoir 110 contient quant à lui du fluide de stockage à une température homogène intermédiaire par rapport à celles des réservoirs 120 et 140. À titre d'exemple, le réservoir 130 est à une température quasi homogène de 45°C, le réservoir 110 est à une température quasi homogène de 65°C, le réservoir 120 est à une température quasi homogène de 85°C

La charge du module de stockage 100 débute par une étape de soutirage du fluide de stockage en prélevant dans un réservoir ayant une température la plus élevée. A la figure 4, en début de charge le réservoir 120 est initialement à la température quasi homogène la plus élevée des trois réservoirs 110, 120, 140 remplis.

Le soutirage débute par un débit de fluide de stockage nécessaire pour assurer le refroidissement du stérilisateur 200 conformément à une consigne de température prédéfinie. Simultanément à l'étape de soutirage débute l'étape d'injection. Ce débit de fluide de stockage qui est réchauffé au cours de son passage dans l'échangeur thermique 102 couplé au stérilisateur 200 est ensuite injecté dans le volume tampon ici, le réservoir 130 avantageusement initialement vide. Le remplissage du réservoir 130 s'effectue par sa partie inférieure avantageusement par le système d'injection et de soutirage 108 et en passant préférentiellement si présent, par un système permettant de répartir et d'uniformiser le débit sur toute la section hydraulique du réservoir. La charge du réservoir 130 par le bas permet de stratifier en température le fluide de stockage injecté, en conservant en partie supérieure le fluide de stockage le plus chaud issu du début du refroidissement du stérilisateur 200 et en partie basse le fluide de stockage le plus froid injecté avantageusement jusqu'au remplissage complet de ce réservoir 130. Lorsque le réservoir 120 est vide ou lorsque sa température interne ne permet plus d'assurer un refroidissement suffisant du stérilisateur 200 au débit maximum admissible dans le réseau de stockage, un autre réservoir dont le fluide de stockage est à une température inférieure à celle du fluide de stockage des réservoirs 120 et 130 est utilisé. En figure 4, c'est le réservoir 110 qui prend le relai. Si le réservoir 130 est plein, le débit de fluide de stockage réchauffé est injecté dans le volume tampon, c'est-à-dire le réservoir avantageusement vide, le réservoir 120, en procédant de la même manière que pour le remplissage du réservoir 130, de sorte à maitriser une stratification thermique du fluide de stockage remplissant le réservoir. La fin de la charge du module de stockage 100 se termine ensuite par le remplissage stratifié en température du réservoir 110, en soutirant dans le réservoir 140 avantageusement initialement à une température homogène. Lors de la phase de charge du module de stockage 100, la sélection des réservoirs 110, 120, 130, 140 débités et des réservoirs alimentés s'effectue par l'intermédiaire les vannes d'isolement des réservoirs, avantageusement pilotables notamment par un module de gestion. Lorsque l'intégralité du module de stockage 100 est chargée ou lorsque son état ne permet plus d'assurer le refroidissement du stérilisateur 200, les vannes d'isolement du circuit de stockage 101 V_{stack,M-iso}, isolent le circuit de stockage 101 de l'échangeur thermique 102 couplé au stérilisateur 200, avant que le réseau d'une autre source froide telle que de l'eau de refroidissement prenne le relai par l'arrivée 302.

Avantageusement, l'étape de soutirage dans les réservoirs 110, 120, 130, 140 initialement à une température homogène différente, s'effectue toujours de préférence en commençant par celui qui est à la température la plus élevée puis s'enchaîne de manière décroissante jusqu'au réservoir qui est à la température la plus faible. Si la température homogène interne du réservoir 110, 120, 130, 140 déchargée est trop importante pour assurer le refroidissement du stérilisateur 200, le réservoir suivant par ordre décroissant de température prend le relai.

A la fin de l'étape de charge, illustrée à la figure 3, trois réservoirs 110, 120, 130 sont remplis de fluide de stockage stratifié en température. À titre d'exemple, le réservoir 110 contient du fluide de stockage présentant du bas vers le haut une température de 45°C à 65°C, le réservoir 120 contient du fluide de stockage présentant du bas vers le haut une température de 65°C à 85°C, le réservoir 130 contient du fluide de stockage présentant du bas vers le haut une température de 85°C à 110°C. Ces conditions correspondent à un cas optimal de fonctionnement ayant conduit à la décharge complète de chacun des réservoirs initialement chargés. Dans un cas moins favorable, la température du fluide de stockage stocké dans un ou plusieurs des réservoirs ne permet pas leur décharge complète. Le réservoir considéré comme tampon pour la décharge suivante ne sera pas initialement entièrement vide. Ce mode peut être qualifié de mode dégradé.

Exemple de décharge du module de stockage 100:
Compte tenu de l'état final de la charge du module de stockage 100 décrite précédemment, illustré en figure 3 qui constitue l'état initial de la décharge, le réservoir 110 est le premier à être déchargé, sous réserve que la température du fluide de stockage soutiré par sa partie inférieure soit suffisante pour suivre la consigne de température imposée pour le fluide de stérilisation en sortie du stérilisateur 200 T_{stw,to}. Dans le cas contraire, la décharge commence par le réservoir suivant qui satisfait cette condition sur la température, et ce par ordre croissant de température. En considérant que la condition sur la température du réservoir 110 est remplie, le fluide de stockage alimente l'échangeur thermique 102 avant d'être injecté, refroidi, dans le volume tampon c'est à dire un réservoir avantageusement vide, en l'espèce le réservoir 140 initialement vide. L'injection du fluide de stockage refroidi dans le réservoir 140 s'opère par la partie inférieure, possiblement par le système d'injection et de soutirage 108 utilisé pour le soutirage. En passant préférentiellement si présent, par un système permettant de répartir et d'uniformiser le débit sur toute la section hydraulique du réservoir. Lors de cette phase de décharge du module de stockage 100, le fluide de stockage injecté dans les réservoirs arrive de plus en plus chaud. Étant donné que l'injection du fluide de stockage dans les réservoirs s'effectue par la partie inférieure, des mouvements de convection naturelle s'amorcent dans le réservoir alimenté compte tenu du gradient de température et de la différence de masse volumique entre le fluide de stockage présent et le fluide de stockage injecté, conduisant à homogénéiser progressivement leur température. Dès la vidange complète du réservoir 110 ou suite à l'arrêt de son soutirage anticipé pour respecter le suivi de la consigne sur la température du fluide de stérilisation envoyé au stérilisateur 200, le réservoir 120 prend le relai. Le débit de fluide de stockage soutiré dans le réservoir 120 et refroidi après son passage dans l'échangeur thermique 102 alimente ensuite le réservoir 110. Le mode opératoire s'enchaîne ensuite lors de cette phase de valorisation de l'énergie thermique stockée jusqu'à la décharge si possible complète du réservoir 130 qui contient un volume de fluide de stockage stratifié au plus haut niveau de température. Dans leurs états finaux illustrés à la figure 4, les réservoirs 110, 120, 140 remplis à la fin de l'étape de décharge du module de stockage 100 s'échelonnent à différents niveaux de températures homogènes.

La figure 6 illustre un schéma de tuyauterie et d'instrumentation (P&ID) comprenant un système de pressurisation des réservoirs qui sont mis sous pression par un gaz et fonctionnant comme cela est décrit en référence à la figure 5. La partie droite du schéma est identique aux figures 1 à 5 avec un stérilisateur 200. L'unité de valorisation selon la figure 6 comprend une pompe 107 équipée d'un variateur de fréquence sur lequel il est possible d'agir de façon à piloter le débit de fluide de stockage circulant dans le circuit de stockage et ce en faisant varier la vitesse de rotation de la pompe. L'unité de valorisation de la figure 6 ne comprend pas de débitmètre. L'unité de valorisation comprend comme module de gestion PID connectant la sonde de température T_{stock - from 1}, les vannes V_{stockM-iso}, et éventuellement l'appoint d'eau. La régulation du débit du fluide caloporteur circulant dans l'unité de valorisation est assurée en agissant sur le variateur de vitesse de la pompe 107, dont le pilotage est géré par le PID en fonction de l'acquisition de la mesure de la température T_{stw, to} et du débit par le débitmètre 109.

La figure 7 illustre un schéma de tuyauterie et d'instrumentation (P&ID) comprenant un système de pressurisation des réservoirs qui sont mis sous pression par un gaz et fonctionnant comme cela est décrit en référence à la figure 5. La partie droite du schéma est identique aux figures 1 à 5 avec le stérilisateur 200. L'unité de valorisation selon la figure 7 comprend une pompe 107 et un débitmètre 109. L'unité de valorisation comprend une vanne VSV-Q destinée à la régulation du débit et avantageusement agencée en amont du débitmètre 109 et en aval de la pompe 107. L'unité de valorisation comprend comme module de gestion PID connectant la pompe 107, la vanne V_{stockM-iso}, le débitmètre 109 et éventuellement l'appoint d'eau. La régulation du débit du fluide caloporteur circulant dans l'unité de valorisation est assurée par la vanne de régulation VSV-Q dont le pilotage est géré par le PID de l'acquisition de la mesure de la température T_{stw, to} et du débit par le débitmètre 109.

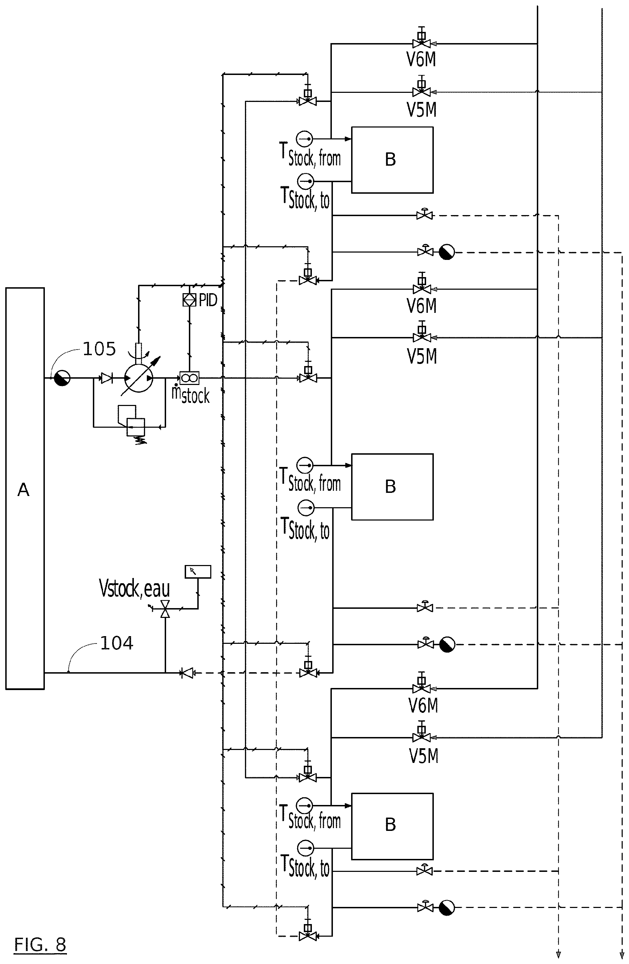

La figure 8 illustre un schéma de tuyauterie et d'instrumentation (P&ID) similaire à la figure 7 sur lequel le dispositif de stérilisation comprend une unité de valorisation comprenant trois échangeurs thermiques 102 chacun respectivement associé à un stérilisateur 200. Les trois échangeurs thermiques 102 sont agencés en parallèle, sur la deuxième branche 105 et sur la première branche 104.

L'invention n'est pas limitée aux modes de réalisations précédemment décrits et s'étend à tous les modes de réalisation couverts par les revendications.

### LISTE DES REFERENCES

11. Première conduite
12. Deuxième conduite
100. Module de stockage
101. Circuit de stockage
102. Echangeur thermique
103. Appoint d'eau
104. Première branche
105. Deuxième branche
106. Branche de dérivation
107. Pompe
108. Système d'injection et de soutirage
109. Débitmètre
110. Réservoir
120. Réservoir
130. Réservoir
140. Réservoir
200. Stérilisateur
201. Circuit de stérilisation
202. Pompe
203. Débitmètre
204. Entrée du fluide de stérilisation
205. Sortie du fluide de stérilisation
206. Entrée d'une source chaude ou froide
207. Sortie d'une source chaude ou froide
301. Arrivée de vapeur
302. Arrivée d'eau de refroidissement
303. Retour eau de refroidissement
304. Retour condensat
400. Module de pressurisation

## Revendications

1. Unité de valorisation comprenant un module de stockage (100) de l'énergie thermique recevant un fluide de stockage, un échangeur thermique (102) et un circuit de stockage thermique (101) configuré pour relier fluidiquement le module de stockage (100) et l'échangeur thermique (102) et assurer la circulation du fluide de stockage auquel est relié le module de stockage (100),
où le module de stockage comprend un volume vide dit volume tampon et en ce que le fluide de stockage destiné à être stocké dans le module de stockage (100) représente un volume inférieur au volume du module de stockage (100) de sorte à maintenir un volume vide dit volume tampon, le module de stockage d'énergie (100) comprend au moins deux réservoirs de stockage de chaleur sensible, dont au moins un premier réservoir (110) et un deuxième réservoir (120) et un système d'injection et de soutirage du fluide de stockage (108) agencé en partie inférieure de chacun des au moins deux réservoirs (110, 120) pour prélever le fluide de stockage en partie inférieure de chacun des au moins deux réservoirs (110, 120) et injecter le fluide de stockage en partie inférieure de chacun des au moins deux réservoirs (110, 120), le système d'injection et de soutirage du fluide de stockage (108) étant configuré pour favoriser la formation et le maintien d'une stratification en température, **caractérisé en ce**
**que** l'unité de valorisation comprend un module de gestion du module de stockage (100) configuré pour contrôler le système d'injection ou de soutirage du fluide de stockage en fonction d'une consigne de température dans l'échangeur thermique (102).

2. Unité de valorisation selon la revendication précédente dans laquelle le volume tampon correspond au volume d'au moins un réservoir (110, 120).

3. Unité de valorisation selon l'une quelconque des revendications précédentes dans laquelle le circuit de stockage thermique (101) est configuré pour fonctionner en circuit fermé

4. Unité de valorisation selon l'une quelconque des revendications précédentes dans laquelle le module de stockage (100) comprend un système d'homogénéisation et de répartition du débit du fluide de stockage injecté dans toute la section hydraulique des au moins deux réservoirs (110, 120).

5. Unité de valorisation selon l'une quelconque des revendications précédentes comprenant un module de pressurisation (400) des au moins deux réservoirs (110, 120) de stockage configuré pour maintenir une pression suffisante pour le maintien du fluide de stockage à l'état liquide.

6. Unité de valorisation selon l'une quelconque des revendications précédentes dans laquelle le module de stockage (100) comprend quatre réservoirs (110, 120, 130, 140).

7. Dispositif de stérilisation comprenant un stérilisateur (200) et une unité de valorisation selon l'une quelconque des revendications précédentes.

8. Dispositif de stérilisation selon la revendication précédente comprenant une arrivée de source froide (302) différente du fluide de stockage et une arrivée de source chaude (301) différente du fluide de stockage.

9. Procédé de valorisation d'énergie thermique par une unité de valorisation selon l'une quelconque des revendications 1 à 6 comprenant une étape de charge du module de stockage (100) comprenant,
- Le soutirage du fluide de stockage en partie inférieure d'un réservoir de stockage, parmi les au moins deux réservoirs de stockage (110, 120, 130, 140), par le circuit de stockage (101) pour échanger l'énergie thermique dans l'échangeur thermique (102),
- L'injection du fluide de stockage en partie inférieure dans le volume tampon,
- le contrôle du système d'injection ou de soutirage du fluide de stockage en fonction d'une consigne de température dans l'échangeur thermique (102) par le module de gestion du module de stockage (100) et/ou
comprenant une étape de décharge du fluide de stockage comprenant,
- Le soutirage du fluide de stockage en partie inférieure d'un réservoir (110, 120, 130, 140) de stockage, parmi les au moins deux réservoirs (110, 120, 130, 140) de stockage par le circuit de stockage (101) pour échanger l'énergie thermique dans l'échangeur thermique (102),
- L'injection du fluide de stockage en partie inférieure dans le volume tampon,
- le contrôle du système d'injection ou de soutirage du fluide de stockage en fonction d'une consigne de température dans l'échangeur thermique (102) par le module de gestion du module de stockage (100).

10. Procédé selon la revendication précédente dans lequel au cours de l'étape de soutirage de l'étape de charge, le réservoir (110, 120, 130, 140) duquel est soutiré le fluide de stockage comprend le fluide de stockage à une température quasi homogène.

11. Procédé selon l'une quelconque des deux revendications précédentes dans lequel au cours de l'étape d'injection de l'étape de charge, le volume tampon est un réservoir (110, 120, 130, 140) différent du réservoir de stockage (110, 120, 130, 140) duquel le fluide de stockage est soutiré, le fluide stockage est injecté en partie inférieure du réservoir (110, 120, 130, 140) et arrive de plus en plus froid assurant une stratification thermique du fluide de stockage dans le volume tampon.

12. Procédé de valorisation d'énergie thermique selon l'une quelconque des trois revendications précédentes dans lequel au cours de l'étape de soutirage de l'étape de décharge, le réservoir (110, 120, 130, 140) duquel est soutiré le fluide de stockage comprend le fluide de stockage stratifié en température, le soutirage étant réalisé en partie inférieure, le prélèvement commence par le fluide de stockage le plus froid jusqu'au plus chaud.

13. Procédé selon l'une quelconque des quatre revendications précédentes dans lequel au cours de l'étape d'injection de l'étape de décharge, le volume tampon est un réservoir (110, 120, 130, 140) différent du réservoir de stockage (110, 120, 130, 140) duquel le fluide de stockage est soutiré, le fluide stockage est injecté en partie inférieure du réservoir (110, 120, 130, 140) et arrive de plus en plus chaud et est mélangé avec le fluide de stockage contenu plus froid.

## Patentansprüche

1. Verwertungseinheit, umfassend ein Speichermodul (100) für Wärmeenergie, das ein Speicherfluid aufnimmt, einen Wärmetauscher (102) und einen Wärmespeicherkreislauf (101), der konfiguriert ist, um das Speichermodul (100) und den Wärmetauscher (102) strömungstechnisch zu verbinden und für die Zirkulation des Speicherfluids, mit dem das Speichermodul (100) verbunden ist, zu sorgen, wobei das Speichermodul ein Puffervolumen genanntes Leervolumen umfasst, und dadurch, dass das Speicherfluid, das dazu bestimmt ist, in dem Speichermodul (100) gespeichert zu werden, ein geringeres Volumen als das Volumen des Speichermoduls (100) darstellt, um ein Puffervolumen genanntes Leervolumen aufrecht zu erhalten, wobei das Energiespeichermodul (100) mindestens zwei Behälter zum Speichern von fühlbarer Wärme umfasst, darunter mindestens einen ersten Behälter (110) und einen zweiten Behälter (120) und ein System zum Einspeisen und Abziehen des Speicherfluids (108), das im unteren Teil eines jeden der mindestens zwei Behälter (110, 120) angeordnet ist, um Speichefluid im unteren Teil eines jeden der mindestens zwei Behälter (110, 120) abzuziehen, und das Speicherfluid im unteren Teil eines jeden der mindestens zwei Behälter (110, 120) einzuspeisen, wobei das System zum Einspeisen und Abziehen des Speicherfluids (108) konfiguriert ist, um die Bildung und die Beibehaltung einer Temperaturschichtung zu fördern, **dadurch gekennzeichnet, dass** die Verwertungseinheit ein Modul zum Verwalten des Speichermoduls (100) umfasst, das konfiguriert ist, um das System zum Einspeisen und Abziehen des Speicherfluids in Abhängigkeit von einem Temperatursollwert im Wärmetauscher (102) zu kontrollieren.

2. Verwertungseinheit nach dem vorstehenden Anspruch, wobei das Puffervolumen dem Volumen von mindestens einem Behälter (110, 120) entspricht.

3. Verwertungseinheit nach einem der vorstehenden Ansprüche, wobei der Wärmespeicherkreislauf (101) konfiguriert ist, um im geschlossenen Kreis zu arbeiten.

4. Verwertungseinheit nach einem der vorstehenden Ansprüche, wobei das Speichermodul (100) ein System zum Vereinheitlichen und Verteilen des Durchsatzes des Speicherfluids, das in den gesamten Hydraulikabschnitt der mindestens zwei Behälter (110, 120) eingespeist wird, umfasst.

5. Verwertungseinheit nach einem der vorstehenden Ansprüche, die ein Druckbeaufschlagungsmodul (400) der mindestens zwei Behälter (110, 120) zum Speichern umfasst, das konfiguriert ist, um einen ausreichenden Druck zum Beibehalten des Speicherfluids im flüssigen Zustand beizubehalten.

6. Verwertungseinheit nach einem der vorstehenden Ansprüche, wobei das Speichermodul (100) vier Behälter (110, 120, 130, 140) umfasst.

7. Sterilisierungsvorrichtung, die einen Sterilisator (200) und eine Verwertungseinheit nach einem der vorstehenden Ansprüche umfasst.

8. Sterilisierungsvorrichtung nach dem vorstehenden Anspruch, die eine Zuleitung einer sich von dem Speicherfluid unterscheidenden Kältequelle (302) und eine Zuleitung einer sich von dem Speicherfluid unterscheidenden Wärmequelle (301) umfasst.

9. Verfahren zum Verwerten von Wärmeenergie durch eine Verwertungseinheit nach einem der Ansprüche 1 bis 6, das einen Ladeschritt des Speichermoduls (100) umfasst, der umfasst
- das Abziehen des Speicherfluids im unteren Teil eines Speicherbehälters aus den mindestens zwei Speicherbehältern (110, 120, 130, 140) durch den Speicherkreislauf (101) zum Austauschen von Wärmeenergie in dem Wärmetauscher (102),
- das Einspeisen des Speicherfluids im unteren Teil in das Puffervolumen,
- das Kontrollieren des Systems zum Einspeisen und Abziehen des Speicherfluids in Abhängigkeit von einem Temperatursollwert im Wärmetauscher (102) durch das Modul zum Verwalten des Speichermoduls (100), und/oder
umfassend einen Entladeschritt des Speicherfluids, der umfasst
- das Abziehen des Speicherfluids im unteren Teil eines Speicherbehälters (110, 120, 130, 140) aus den mindestens zwei Speicherbehältern (110, 120, 130, 140) durch den Speicherkreislauf (101) zum Austauschen von Wärmeenergie in dem Wärmetauscher (102),
- das Einspeisen des Speicherfluids im unteren Teil in das Puffervolumen,
- das Kontrollieren des Systems zum Einspeisen und Abziehen des Speicherfluids in Abhängigkeit von einem Temperatursollwert im Wärmetauscher (102) durch das Modul zum Verwalten des Speichermoduls (100).

10. Verfahren nach dem vorstehenden Anspruch, wobei im Laufe des Schrittes des Abziehens des Ladeschrittes der Behälter (110, 120, 130, 140), aus dem das Speicherfluid abgezogen wird, das Speicherfluid auf einer quasi homogenen Temperatur umfasst.

11. Verfahren nach einem der beiden vorstehenden Ansprüche, wobei im Laufe des Schrittes des Einspeisens des Ladeschrittes das Puffervolumen ein anderer Behälter (110, 120, 130, 140) als der Speicherbehälter (110, 120, 130, 140) ist, aus dem das Speicherfluid abgezogen wird, das Speicherfluid in den unteren Teil des Behälters (110, 120, 130, 140) eingespeist wird, und immer kälter ankommt, wodurch für eine Wärmeschichtung des Speicherfluids im Puffervolumen gesorgt wird.

12. Verfahren zum Verwerten von Wärmeenergie nach einem der drei vorstehenden Ansprüche, wobei im Laufe des Schrittes des Abziehens des Entladeschrittes der Behälter (110, 120, 130, 140), aus dem das Speicherfluid abgezogen wird, das temperaturgeschichtete Speicherfluid umfasst, wobei das Abziehen im unteren Teil erfolgt, die Entnahme mit dem kältesten Speicherfluid bis zum wärmsten beginnt.

13. Verfahren nach einem der vier vorstehenden Ansprüche, wobei im Laufe des Schrittes des Einspeisens des Entladeschrittes das Puffervolumen ein anderer Behälter (110, 120, 130, 140) als der Speicherbehälter (110, 120, 130, 140) ist, aus dem das Speicherfluid abgezogen wird, das Speicherfluid in den unteren Teil des Behälters (110, 120, 130, 140) eingespeist wird, und immer wärmer ankommt, und mit dem enthaltenen kälteren Speicherfluid gemischt wird.

## Claims

1. A reclaiming unit comprising a heat energy storage module (100) receiving a storage fluid, a heat exchanger (102) and a heat storage circuit (101) configured to fluidly connect the storage module (100) and the heat exchanger (102) and to ensure circulation of the storage fluid to which the storage module (100) is connected, wherein the storage module comprises an empty so-called buffer volume and in that the storage fluid to be stored in the storage module (100) represents a volume smaller than the volume of the storage module (100) so as to keep an empty so-called buffer volume, the energy storage module (100) comprises at least two sensible heat storage reservoirs, including at least a first reservoir (110) and a second reservoir (120), and a storage fluid injection and withdrawal system (108) arranged in the lower part of each of the at least two reservoirs (110, 120) for removing the storage fluid from the lower part of each of the at least two reservoirs (110, 120) and injecting the storage fluid into the lower part of each of the at least two reservoirs (110, 120), the storage fluid injection and withdrawal system (108) being configured to promote formation and maintenance of temperature stratification, **characterised in that** the reclaiming unit comprises a storage module management module (100) configured to control the storage fluid injection or withdrawal system as a function of a temperature set point in the heat exchanger (102).

2. The reclaiming unit according to the preceding claim, wherein the buffer volume corresponds to the volume of at least one reservoir (110, 120).

3. The reclaiming unit according to any of the preceding claims, wherein the heat storage circuit (101) is configured to operate as a closed circuit.

4. The reclaiming unit according to any of the preceding claims, wherein the storage module (100) comprises a system for homogenising and distributing flow rate of the storage fluid injected throughout the hydraulic section of the at least two reservoirs (110, 120).

5. The reclaiming unit according to any of the preceding claims, comprising a pressurisation module (400) for the at least two storage reservoirs (110, 120) configured to keep a pressure sufficient to keep the storage fluid in the liquid state.

6. The reclaiming unit according to any of the preceding claims, wherein the storage module (100) comprises four reservoirs (110, 120, 130, 140).

7. A sterilisation device comprising a steriliser (200) and a reclaiming unit according to any of the preceding claims.

8. The sterilisation device according to the preceding claim, comprising a cold source inlet (302) different from the storage fluid and a hot source inlet (301) different from the storage fluid.

9. A method for reclaiming heat energy by a reclaiming unit according to any of claims 1 to 6, comprising a step of loading the storage module (100) comprising,
- withdrawing the storage fluid from the lower part of a storage reservoir, from among the at least two storage reservoirs (110, 120, 130, 140), through the storage circuit (101) in order to exchange heat energy in the heat exchanger (102),
- injecting the storage fluid at the lower part into the buffer volume,
- controlling the storage fluid injection or withdrawal system as a function of a temperature set point in the heat exchanger (102) by the management module of the storage module (100) and/or
comprising a storage fluid unloading step comprising
- withdrawing the storage fluid from the lower part of a storage reservoir (110, 120, 130, 140), from among the at least two storage reservoirs (110, 120, 130, 140) via the storage circuit (101) in order to exchange heat energy in the heat exchanger (102),
- injecting the storage fluid at the lower part into the buffer volume,
- controlling the storage fluid injection or withdrawal system as a function of a temperature set point in the heat exchanger (102) by the management module of the storage module (100).

10. The method according to the preceding claim, wherein during the withdrawal step of the loading step, the reservoir (110, 120, 130, 140) from which the storage fluid is withdrawn comprises the storage fluid at a quasi-homogeneous temperature.

11. The method according to any of the two preceding claims, wherein during the injection step of the loading step, the buffer volume is a reservoir (110, 120, 130, 140) different from the storage reservoir (110, 120, 130, 140) from which the storage fluid is withdrawn, the storage fluid is injected into the lower part of the reservoir (110, 120, 130, 140) and arrives colder and colder, ensuring thermal stratification of the storage fluid in the buffer volume.

12. The method for reclaiming heat energy according to any of the three preceding claims, wherein, during the withdrawal step of the unloading step, the reservoir (110, 120, 130, 140) from which the storage fluid is withdrawn comprises the temperature-stratified storage fluid, the withdrawal being carried out at the lower part, the withdrawal beginning with the coldest to the warmest storage fluid.

13. The method according to any of the four preceding claims, wherein, during the injection step of the unloading step, the buffer volume is a reservoir (110, 120, 130, 140) different from the storage reservoir (110, 120, 130, 140) from which the storage fluid is withdrawn, the storage fluid is injected into the lower part of the reservoir (110, 120, 130, 140) and arrives increasingly warmer and is mixed with the colder contained storage fluid.
